# EUROPEAN PATENT APPLICATION

(11) **EP 3 165 611 A1**
(43) Date of publication of application: **10.05.2017**
(21) Application number: 15791978.8
(22) Date of filing: 18.05.2015
(51) Int. Cl.: C12Q 1/68, C12N 15/12

(54) **USE OF POLYMORPHISMS OF THE GENE NFKB1 FOR AUDITORY PROGNOSIS FOR MENIÈRE'S DISEASE**

(30) Priority: 16.05.2014 ES 201430716
(71) Applicant: Agencia Pública Empresarial Sanitaria Hospital de Poniente, 04700 El Ejido (Almería) (ES); Servicio Andaluz de Salud, 41071 Sevilla (ES); Fundación Pública Andaluza Progreso Y Salud, 41092 Sevilla (ES); Icahn School of Medicine Mount Sinai, New York, NY 10029 (US)
(72) Inventor: LÓPEZ ESCÁMEZ, José Antonio, 04700 El Ejido (Almería) (ES); CABRERA MARTÍNEZ, Sonia, 41092 Sevilla (ES); ALARCÓN RIQUELME, Marta Eugenia, 41092 Sevilla (ES); REQUENA NAVARRO, María Teresa, 41092 Sevilla (ES); ESPINOSA SÁNCHEZ, Juan Manuel, 41071 Sevilla (ES); SÁNCHEZ RODRÍGUEZ, Elena, New York, NY 10029 (US)
(74) Representative: Hoffmann Eitle
(86) International application number: PCT/ES2015/070392
(87) International publication number: WO 2015/173461

(57) **Abstract**

The present invention describes the use of a group of single-nucleotide polymorphisms (SNPs) or variants in the gene *NFKB1* in order to obtain data useful for the prognosis of a disease occurring with sensorineural hearing loss, and to the kit or devices and uses thereof.

SEQUENCE LISTING

<110>  AGENCIA PUBLICA EMPRESARIAL SANITARIA HOSPITAL DE PONIENTE
       SERVICIO ANDALUZ DE SALUD
       FUNDACION PUBLICA ANDALUZA PROGRESO Y SALUD
       MOUNT SINAI HOSPITAL CORP

<120>  Uso de polimorfismos del gen NFKB1 para el pronóstico auditivo en
        la enfermedad de Menière.

<130>  FIBAO-14010

<160>  2

<170>  PatentIn version 3.5

<210>  1
<211>  51
<212>  DNA
<213>  Homo sapiens

<400>  1
tatcaaaatt aaaattctgt gatacyatgt ttttagggaa gaagaaaact g          51

<210>  2
<211>  51
<212>  DNA
<213>  Homo sapiens

<400>  2
ctgaaaattt gcagacttca ggctakgcct gtaaataaat aaataagaag t          51

## Description

The present invention is encompassed within the field of biomedicine and biotechnology, and relates to a method for obtaining data useful for the prognosis and classification of patients suffering from sensorineural hearing loss, and particularly those having Ménière's disease.

### PRIOR STATE OF THE ART

Ménière's disease (MD) is a chronic disorder affecting the inner ear characterized by recurrent episodes of vertigo, progressive instability, pressure in the ears, tinnitus and hearing loss. An autoimmune etiology has been proposed for this disease given the observation of an increased number of autoantibodies and a clinical improvement with steroid therapy. Some genetic variants in genes of the immune system such as *MICA* or *TLR10* seem to modify the clinical course of the disease, possibly as a result of a more efficient immune response.

Human major histocompatibility complex class I and class II molecules have been described as genetic markers for many autoimmune diseases. In turn, MD has been associated with *HLA-B*27, HLA-B*44, HLA*-*B***13*, *HLA-Cw*07, HLA-DBR1*1602* and *HLA-DRB1*1101* in different populations, however these associations are not useful in diagnostics.

Some research studies have suggested an association between the gene *TLR10* and autoimmune diseases. On the other hand, the product of the gene *TLR10* is an unknown ligand receptor that may play an important role in the autoimmune process and the exon 3 variants of this gene can modulate innate immune response and determine the condition of both ears. Therefore, systematic study by means of a genotyping microarray for genotyping variants previously associated with other autoimmune diseases in 186 loci (immunochip) seems to be a way to explore the maximum number of variants per sample, which improves diagnostic yield.

A wide range of medical and surgical treatments for hearing loss, and specifically for Ménière's disease, has been proposed. All of them have yielded different results with respect to symptom remission and side effects.

Medical treatments include, among others, a low-salt diet, antihistamines, diuretics, subcutaneous histamine, antivertigo drugs, benzodiazepines and transtympanic therapy, which has gained certain popularity in recent times, particularly in the group of patients who do not respond to conventional therapies
Some centers have for some time now performed intratympanic injection of ototoxic medicinal products, such as gentamicin, into the middle ear cavity. The purpose of this procedure would be to achieve medical labyrinthectomy which would cause the reduction or disappearance of symptoms. The risk of this procedure is the deterioration of the patient's hearing ability, particularly described in those patients subjected to short-term treatment regimens. The most widely used agents were, at first, streptomycin and, now gentamicin.

Surgery was resorted to in very severe cases, but this resort worsens the patient's quality of life because it eliminates not only symptoms, but also sense of balance.

Therefore, currently available treatments for hearing loss, such as ablative surgery (vestibular neurectomy or labyrinthectomy) or intratympanic gentamicin application, for example, can be extremely aggressive and can put the patient's hearing at risk. It would be important to provide a prognosis marker which will allow evaluating treatment suitability.

However, up until now there has been no available biological method or marker which allows early diagnosis or establishing a long-term hearing loss prognosis which is useful for patient classification and makes it easier to make decisions concerning treatment and disease progression follow-up.

### BRIEF DESCRIPTION OF THE INVENTION

The examples of the invention show that single-nucleotide variants rs3774937 and rs4648011 are markers useful for determining the prognosis of a disease occurring with sensorineural hearing loss, preferably for the prognosis of Ménière's disease with unilateral sensorineural hearing loss, as well as for evaluating the genetic risk of developing hearing loss >40 dB in the early years of the disease.

Therefore, a first aspect of the invention relates to the use of the gene *NFKB1* for obtaining data useful in a disease occurring with sensorineural hearing loss.

In a preferred embodiment of this aspect of the invention, single-nucleotide polymorphisms or variants rs3774937 and/or rs4648011 of the gene *NFKB1* are used for obtaining data useful for the prognosis of a disease occurring with sensorineural hearing loss.
In another preferred embodiment of this aspect of the invention, the disease occurring with sensorineural hearing loss is Ménière's disease or autoimmune inner ear disease. In another preferred embodiment of this aspect of the invention, the sensorineural hearing loss is unilateral.

A second aspect of the invention relates to a method for obtaining data useful for the prognosis of a disease occurring with sensorineural hearing loss, hereinafter first method of the invention, which comprises:
a) obtaining a biological sample isolated from an individual, and
b) detecting single-nucleotide polymorphisms or variants rs3774937 and/or rs4648011 of the gene *NFKB1* in the isolated biological sample of (a).

Another aspect of the invention relates to a method for the prognosis of a disease occurring with sensorineural hearing loss, hereinafter second method of the invention, which comprises steps (a) and (b) of the first method of the invention, and further comprises:
c) including the individual from (a) in the group of individuals with rapid progression of the disease occurring with sensorineural hearing loss when said individual has a lower homozygous allele frequency for rs3774937 and/or rs4648011.

In a preferred embodiment of this aspect of the invention, the disease occurring with sensorineural hearing loss is Ménière's disease or autoimmune inner ear disease. More preferably, the disease occurring with sensorineural hearing loss is Ménière's disease.

In another preferred embodiment of this aspect the invention, the sample isolated in (a) is peripheral blood, and even more preferably genomic DNA obtained from peripheral blood.

In another preferred embodiment of the invention, the individual belongs to a population of European descent, and even more preferably of Spanish descent.

In another preferred embodiment of this aspect of the invention, the sensorineural hearing loss is unilateral.

A third aspect of the invention relates to a method, hereinafter third method of the invention, for classifying an individual suffering or who has the probability of suffering from a disease occurring with sensorineural hearing loss, preferably Ménière's disease or autoimmune inner ear disease, and more preferably with unilateral sensorineural hearing loss, in one of two groups, wherein group 1 comprises individuals who can be identified by means of the method according to the first and second method of the invention, and wherein group 2 represents the remaining individuals.

A fourth aspect of the invention relates to the use of a pharmaceutical composition comprising gentamicin in the preparation of a medicinal product for the treatment of an individual from group 1 who can be identified by means of the third method of the invention.

A fifth aspect of the invention relates to a prognostic kit or device, hereinafter prognostic kit or device of the invention, comprising at least one oligonucleotide complementary to any of the sequences SEQ ID NO: 1 to SEQ ID NO: 2, or to any of the combinations thereof.

A sixth aspect of the invention relates to a microarray, hereinafter microarray of the invention, comprising oligonucleotides or single-channel microarrays designed based on the nucleotide sequence SEQ ID NO: 1 and/or SEQ ID NO: 2.

A seventh aspect of the invention relates to the use of a kit or a device of the invention or of a microarray of the invention for the prognosis of a disease occurring with sensorineural hearing loss. In a preferred embodiment of this aspect of the invention, the disease occurring with sensorineural hearing loss is Ménière's disease. In another preferred embodiment of this aspect of the invention, the sensorineural hearing loss is unilateral.

Another aspect of the invention relates to a computer-readable storage medium comprising program instructions capable of making a computer run the steps of any of the methods of the invention.

Another aspect of the invention relates to a transmissible signal comprising program instructions capable of making a computer run the steps of any of the methods of the invention.

### DESCRIPTION OF THE DRAWINGS

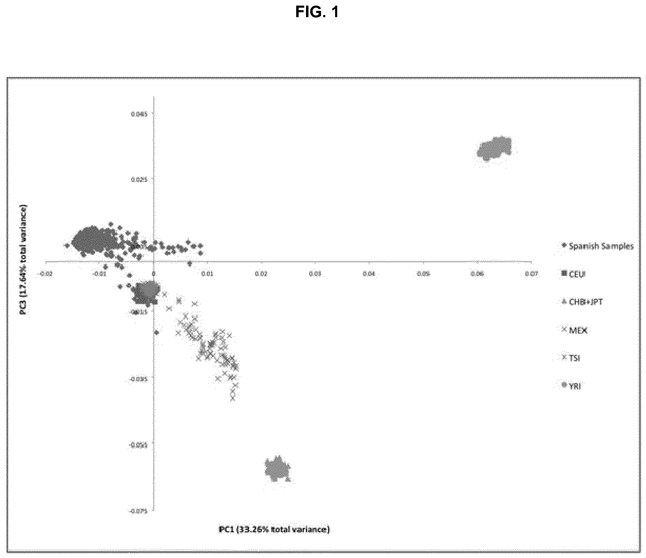

Figure 1 shows a scatter plot showing the principal component analysis (PCA) of the genetic markers in Spanish samples of the present invention in comparison with different HapMap populations. The actual values corresponding to the first three principal components explained a large part of the population substructure in this analysis (77.5%). All the people not included in the main group (>3 standard deviations from the center grouping) were excluded from subsequent analysis. A total of 48 atypical individuals were identified with this method in the cohort of cases and controls of the present invention. The X-axis represents Principal Component 1 (PC1) and the Y-axis represents Principal Components 3 (PC3) in Spanish samples of the present invention (diamonds), and the main HapMap populations: CEU (squares), CHB + JPB (triangles), MEX (crosses), TSI (asterisks) and YRI (circles).
Figure 2 shows variants of the gene *NFKB1* and MD patients' auditory results. A. Carriers of C allele in rs3774937 showed a shorter time to reach hearing stage 3 (>40dB). B. The mean time to reach hearing stage 3 for carriers of G allele in rs4648011 decreased by 2 years (log-rank test, p=0.001 for rs3774937 and p=3.45x10⁻⁴ for rs4648011).
Figure 3 shows a region of the gene *NFKB1* with linkage disequilibrium showing haploblocks with rs3774937 and rs4648011 (r²=0.67, D'=0.95).

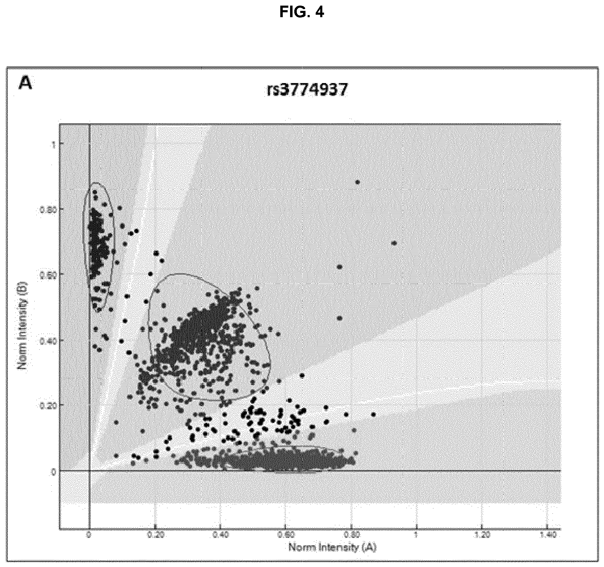

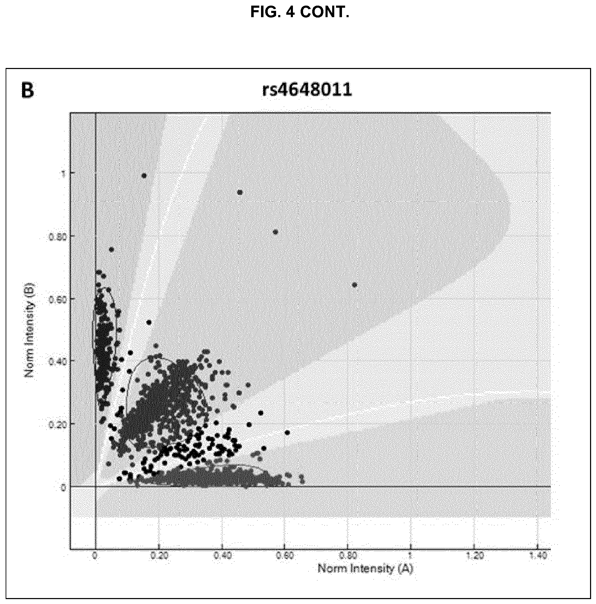

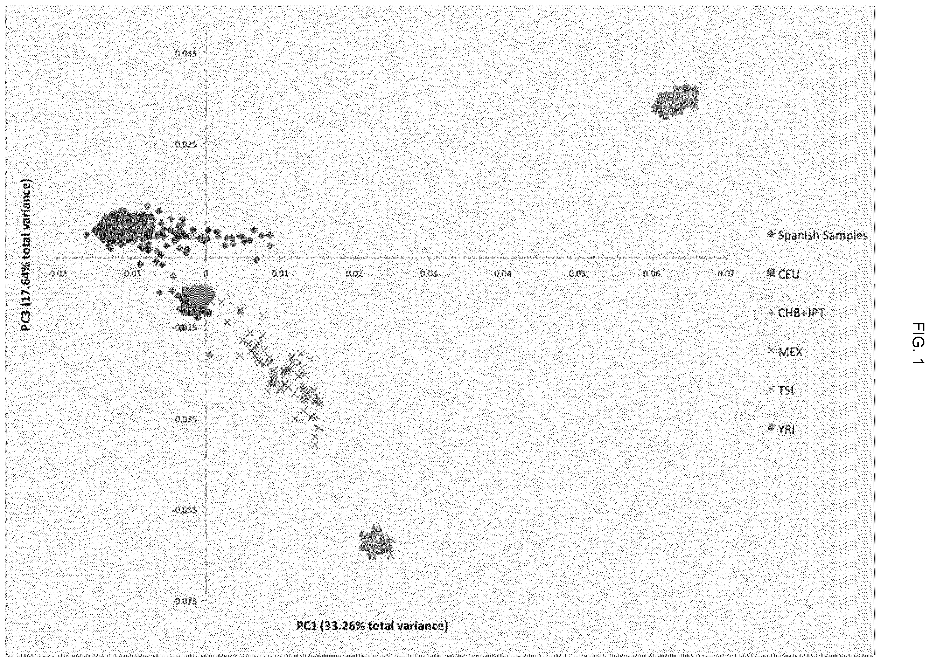

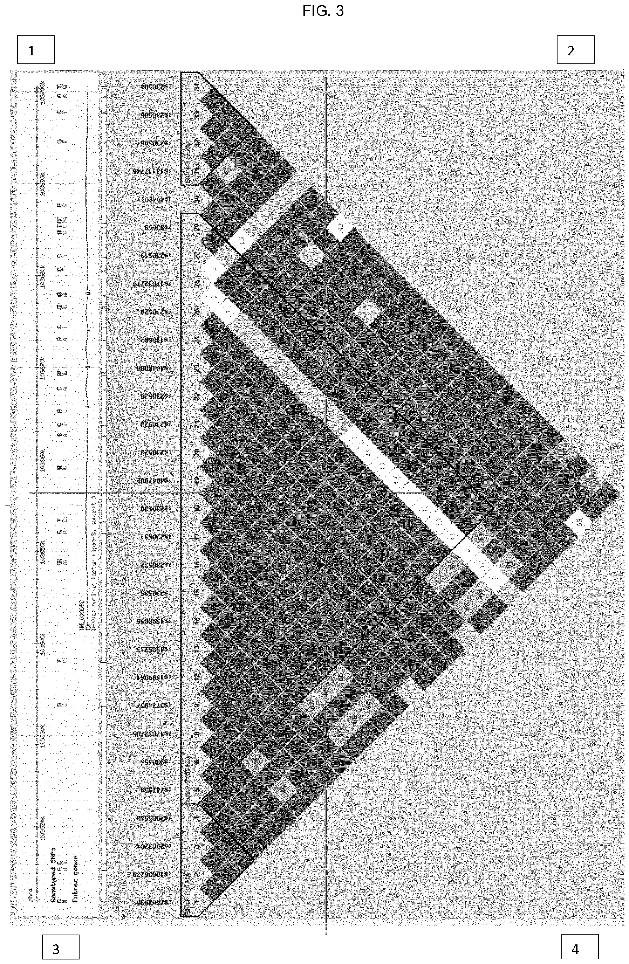

Figure 4 shows the normalized intensity of the signal corresponding to the genotypes of variants rs3774937 and rs4648011 by means of the microarray used in iSCAN.

### DETAILED DESCRIPTION OF THE INVENTION

The present invention provides a method for obtaining data useful for the prognosis of Ménière's disease with unilateral sensorineural hearing loss, as well as for evaluating the genetic risk of developing hearing loss >40 dB in the early years of the disease, which allows optimizing follow-up, treatment planning and genetic counseling. The authors of the present invention are the first to demonstrate the association of two of the allelic variants of the gene *NFKB1* located at specific genomic coordinates which determine a high risk of developing early hearing loss in Ménière's disease with unilateral sensorineural hearing loss. Said variants have a high frequency (0.29 and 0.42) in the general population and in patients and are very useful for establishing the prognosis of the progression of hearing loss with a high diagnostic yield. Therefore, the presence of one of the allelic variants of the gene *NFKB1* in a diagnostic panel determine a high risk of developing early hearing loss for Ménière's disease in the Spanish population and in the population of European descent.

A first aspect of the invention relates to the use of the gene *NFKB1* for obtaining data useful in a disease occurring with sensorineural hearing loss.

In a preferred embodiment of this aspect of the invention, single-nucleotide polymorphisms or variants rs3774937 and rs4648011 of the gene *NFKB1* are used for obtaining data useful for the prognosis of a disease occurring with sensorineural hearing loss.

The gene *NFKB1* encodes a 105 kD protein which is processed in proteosome 26S to produce a 50 kD protein. The 105 kD protein is a specific Rel protein transcription inhibitor and the 50 kD protein is a DNA-binding subunit of the NF-kappa-B (NFKB) protein complex. NFKB is a transcription regulator which is activated by various intra- and extracellular stimuli such as cytokines, free radicals, ultraviolet radiation and bacteria or viral products. Activated NFKB is translocated into the nucleus and stimulates the expression of genes involved in a wide range of biological functions. Anomalous NFKB activation has been associated with various inflammatory and autoimmune diseases. The gene *NFKB1* and its variants are defined in the PubMed database by the reference Gene ID: 4790.

In another preferred embodiment of this aspect of the invention, the disease occurring with sensorineural hearing loss is Ménière's disease or autoimmune inner ear disease. In another preferred embodiment of this aspect of the invention, the sensorineural hearing loss is unilateral.

"Sensorineural hearing loss" is herein understood as the reduction of the hearing level below what is considered normal, so depending on the loss of intensity measured in decibels (dB), hearing loss is classified as:
- Mild hearing loss - when hearing loss is less than 35 dB.
- Moderate hearing loss - when hearing loss is between 35 and 60 dB.
- Profound or severe hearing loss - when hearing loss is between 60 and 90 dB.
- Complete hearing loss or anacusis - when hearing loss is more than 90 dB.

Several methods are known in the state of the art for measuring whether or not there is a loss of hearing level, and the degree of said loss, such as for example, but without limitation, liminal tone audiometry or brainstem auditory evoked potentials.

Furthermore, for Ménière's disease the American Academy of Otolaryngology-Head and Neck Surgery (AAO-HNS) defined in 1995 four hearing stages based on the hearing threshold with 500, 1000, 2000 and 3000 Hz frequencies, where hearing loss is classified as:
- Stage 1: when hearing loss is less than or equal to 25 dB
- Stage 2: when hearing loss is between 26 and 40 dB
- Stage 3: when hearing loss is between 41-70 dB
- Stage 4: when hearing loss is more than 70 dB

It is determined by means of pure-tone audiometry. Hearing loss can occur in one or both ears and can be sudden or abrupt, rapidly progressive (weeks or months) or slowly progressive (months or years). Sensorineural hearing loss affects the inner ear and occurs when the cochlear sensorineural elements or cochlear nerve are injured in some way by physical means or means of a different nature.

"Unilateral sensorineural hearing loss" is herein understood as when the effect occurs only in one ear.

The cochlea is the fundamental auditory organ located in the inner ear, having the shape of a duct wound into a spiral and containing the organ of Corti in which mechanical vibrations are converted into nerve impulses which, through the auditory nerve, reach the brain to be identified. When the cells of the organ of Corti and/or the auditory nerve are affected, sound transmission is interrupted. Any of these delicate structures can be affected by different infectious, inflammatory, toxic or degenerative processes, of all these loud noise and degenerative processes being those responsible for a large part of perceptive deafness.

Depending on the effected organ, perceptive deafness can be classified as:
- Endocochlear: when the inner ear is affected.
- Retrocochlear: when the auditory nerve is affected.

In a preferred embodiment of this aspect of the invention, the sensorineural hearing loss is selected from the list consisting of sudden hearing loss, rapidly progressive hearing loss, slowly progressive hearing loss, low frequency sensorineural hearing loss, immune-mediated inner ear disease, or any of the combinations thereof.

In another preferred embodiment, the sensorineural hearing loss is furthermore associated with recurrent attack of vertigo. In another even more preferred embodiment, the disease associated with sensorineural hearing loss is Ménière's disease. In another more preferred embodiment, the regions of the genome containing the allelic susceptibility variants are those corresponding to the genomic coordinates of Table 1.

**Table 1. Genomic coordinates of the variants forming the Ménière's disease risk diagnostic panel**

| Chromosome | Position in human genome Feb 2009 (GRCh37/hg19) | Allele ⁽¹⁾ | Gene | Location |
|---|---|---|---|---|
| 2 | 103434253 | C/T | *NFKB1* | Intron |
| 2 | 103475444 | G/T | *NFKB1* | Intron |

| | | | | |
|---|---|---|---|---|
| ⁽¹⁾ The first allele is the minor allele and the susceptibility variant, whereas the second allele is the major allele and the reference in the consensus sequence. | | | | |

In the context of the present invention, these variants are also defined by a nucleotide or polynucleotide sequence which is defined in sequences SEQ ID NO: 1, SEQ ID NO: 2, or any of the variants thereof (Table 2).
Chr4: 103434253, rs3774937 = SEQ ID NO: 1
   TATCAAAATTAAAATTCTGTGATAC**[C/T]**ATGTTTTTAGGGAAGAAGAAAACTG
Chr4: 103475444, rs46448011 SEQ ID NO: 2
   CTGAAAATTTGCAGACTTCAGGCTA**[G/T]**GCCTGTAAATAAATAAATAAGAAGT

**Table 2. Identification sequences corresponding to the genomic coordinates of the variants object of the present invention**

| SEQ ID | Chromosome | Position in human genome Feb 2009 (GRCh37/hg19) | Contig | FASTA sequence |
|---|---|---|---|---|
| 1 | 2 | 103434253 | rs3774937 | C/T |
| 2 | 2 | 103475444 | rs46448011 | G/T |

### METHODS OF THE INVENTION

A second aspect of the invention relates to a method for obtaining data useful for the prognosis of a disease occurring with sensorineural hearing loss, hereinafter first method of the invention, which comprises:
a) obtaining a biological sample isolated from an individual, and
b) detecting single-nucleotide polymorphisms or variants rs3774937 and rs4648011 of the gene *NFKB1* in the isolated biological sample of (a).

In other words, step (b) consists of detecting single-nucleotide polymorphisms or variants that are selected from the list consisting of rs3774937 and rs4648011, or any of the combinations thereof. Preferably, all single-nucleotide polymorphisms or variants of the panel of variants of the invention are detected.

Another aspect of the invention relates to a method, hereinafter second method of the invention, for the prognosis of a disease occurring with sensorineural hearing loss which comprises steps (a) and (b) of the first method of the invention, and further comprises:
c) including the individual from (a) in the group of individuals with rapid progression of the disease occurring with sensorineural hearing loss when said individual has a lower homozygous allele frequency for rs3774937 and/or rs4648011.

In other words, they would be the CC genotype for rs3774937 and GG genotype for rs4648011. Although any of the variants described in the present invention or any of the combinations thereof can be used, in a preferred embodiment all the variants or polymorphisms of the invention are used.

The term "rapid progression of the disease" is herein understood as a progression in which the mean time for reaching stage 3 (>40 dB HL) in individuals was reduced by at least 1.5 years.

In a preferred embodiment of this aspect of the invention, the disease occurring with sensorineural hearing loss is Ménière's disease or autoimmune inner ear disease.

An "isolated biological sample" includes, but without limitation, cells, tissues and/or biological fluids from an organism, obtained by means of any method known by a person skilled in the art. Preferably, the isolated biological sample is peripheral blood and/or comprises peripheral blood cells (PBCs), more preferably mononuclear PBCs. More preferably, the isolated sample is genomic DNA, and more preferably obtained from peripheral blood.

In another preferred embodiment of the invention, the individual belongs to a population of European descent, and even more preferably of Spanish descent.

In another preferred embodiment of this aspect of the invention, the sensorineural hearing loss is unilateral.

In a preferred embodiment of this aspect of the invention, the sensorineural hearing loss is selected from the list consisting of sudden hearing loss, rapidly progressive hearing loss, slowly progressive hearing loss, low frequency sensorineural hearing loss, immune-mediated inner ear disease, or any of the combinations thereof. In another preferred embodiment, the sensorineural hearing loss is furthermore associated with recurrent attack of vertigo.

As it is used herein, the term "individual" refers to animals, preferably mammals, and more preferably humans. The term "individual" is not intended to be limiting in any aspect, said individual being able to be of any age and sex and have any physical condition.

In the sense used herein, the term "variant" refers to a sequence substantially homologous to the sequence of the genes containing the described variants. Generally, a variant includes nucleotide additions, deletions or substitutions. More preferably, the described variants refer to the change or substitution of a nucleotide of the sequence in the reference human genome in the described positions.

The term "polymorphism" refers to a variation in the nucleotide sequence of a nucleic acid in which every possible sequence is present in a proportion equal to or greater than 1% in a population; in a particular case, when said variation in the nucleotide sequence occurs in only one nucleotide (A, C, T or G), it is referred to as SNP.

The term "gene mutation" refers to a variation in the nucleotide sequence of a nucleic acid in which every possible sequence is present in a proportion less than 1% in a population.

The terms "allelic variant" or "allele" are used interchangeably herein and refer to a polymorphism occurring in one and the same locus in one and the same population.

As it is used herein, the term "gene variation" or "gene variant" includes mutations, polymorphisms and allelic variants. A gene variation or variant is found among individuals within populations and among populations within species.

The single-nucleotide polymorphisms or variants of the invention can be detected by any method known in the state of the art, for example, but without limitation, by means of DNA sequencing, capillary electrophoresis, mass spectrometry, single-strand conformation polymorphism (SSCP), electrochemical analysis, denaturing HPLC in electrophoresis gel, restriction fragment length polymorphisms (RFLPs) and hybridization analysis in genotyping microarrays.

Steps (b) and/or (c) of the methods described above can be completely or partially automated, for example, by means of robotic sensing equipment for detection in step (b) or computerized comparison in step (c).

As it is used herein, the term "prognosis" refers to the capacity of discriminating between individuals having a higher or lower risk of suffering from severe sensorineural hearing loss in a shorter time period, and more preferably, a higher or lower risk of suffering from Ménière's disease or a higher or lower risk of suffering from autoimmune inner ear disease.

In turn, considering the method of the present invention, other subclassifications can be established within this main classification, therefore making it easier to select and to establish suitable therapeutic regimens. As understood by a person skilled in the art, this discrimination is not intended to be correct for all the analyzed samples. However, it requires a statistically significant amount of the analyzed samples to be correctly classified. The statistically significant amount can be established by a person skilled in the art by means of using different statistical tools, for example, but without limitation, by means of determining confidence intervals, determining significance value P, Student's test or Fisher's discriminant functions, non-parametric Mann Whitney measurements, Spearman's correlation, logistic regression, linear regression, area under the ROC curve (AUC). Preferably, the confidence intervals are at least 90%, at least 95%, at least 97%, at least 98% or at least 99%. Preferably, the p-value is less than 0.1, 0.05, 0.01, 0.005 or 0.0001. Preferably, the present invention allows correctly detecting predisposition to the disease or detecting, in a differential manner, the disease in at least 60%, more preferably in at least 70%, much more preferably in at least 80%, or even much more preferably in at least 90% of the subjects from a specific group or analyzed population.

Another aspect of the invention relates to a method, the third method of the invention, for classifying an individual suffering or who has the probability of suffering from a disease occurring with sensorineural hearing loss, preferably Ménière's disease or autoimmune inner ear disease, and more preferably with unilateral sensorineural hearing loss, in one of two groups, wherein group 1 comprises individuals who can be identified by means of the method according to the first and second method of the invention, and wherein group 2 represents the remaining individuals.

Another aspect of the invention relates to the use of a pharmaceutical composition comprising gentamicin in the preparation of a medicinal product for the treatment of an individual from group 1 who can be identified by the third method of the invention.

### DIAGNOSTIC KIT OR DEVICE AND USES

Another aspect of the invention relates to a kit or device, hereinafter kit or device of the invention, comprising the elements required for detecting single-nucleotide polymorphisms or variants rs3774937 and/or rs4648011 of the gene *NFKB1.*

In a preferred embodiment, the kit or device of the invention comprises primers, probes and/or antibodies capable of detecting sequences SEQ ID NO: 1 and/or SEQ ID NO: 2, and wherein:
- the primers are polynucleotide sequences having between 10 and 30 base pairs, more preferably between 15 and 25 base pairs, even more preferably between 18 and 22 base pairs, and even much more preferably about 20 base pairs and an identity of at least 80%, more preferably of at least 90%, even more preferably of at least 95%, even much more preferably of at least 98%, and particularly of 100%, with a fragment of the sequences complementary to SEQ ID NO: 1 and/or SEQ ID NO: 2;
- the probes are polynucleotide sequences having between 80 and 1100 base pairs, more preferably between 100 and 1000 base pairs, and even more preferably between 200 and 500 base pairs and an identity of at least 80%, more preferably of at least 90%, even more preferably of at least 95%, even much more preferably of at least 98%, and particularly of 100%, with a fragment of the sequences complementary to SEQ ID NO: 1 and/or SEQ ID NO: 2.
- the antibodies are capable of binding specifically to a region formed by any of the amino acid sequences generated from the translation of SEQ NO: 1 and/or SEQ ID NO: 2

In another preferred embodiment of this aspect of the invention, the primers, probes or antibodies are modified or labeled, for example, but without limitation, by means of a radioactive labeling or immunolabeling.

In a preferred embodiment, the kit or device of the invention comprises at least one oligonucleotide complementary to any of the sequences SEQ ID NO: 1 and/or SEQ ID NO: 2.

Said kit or device can contain all those reagents required for analyzing the single-nucleotide polymorphisms or variants of the invention by means of any of the methods existing in the state of the art and/or described herein. The kit can further include, without any type of limitation, buffers, agents to prevent contamination, protein degradation inhibitors, etc. On the other hand, the kit can include all the supports and containers required for operation and optimization. Preferably, the kit further comprises instructions to carry out any of the methods of the invention.

The oligonucleotide/oligonucleotides can also be immobilized in spots on a surface (preferably a solid surface). One of the embodiments of the kit comprises a microarray, or microarray of the invention. An RNA microarray is an array on a solid substrate (usually a glass slide or a silicon thin-film cell) which evaluates large amounts of different RNAs that can be detected by means of specific probes immobilized in spots on a solid substrate. Each spot contains a specific nucleic acid sequence, usually a DNA sequence, as probes (or tracers). Although the number of spots is not in any way limited, there is a preferred embodiment in which the microarray is customized for the methods of the invention. In one embodiment, said customized array comprises fifty spots or less, such as thirty spots or less, including twenty spots or less. Therefore, another aspect of the invention relates to a microarray comprising oligonucleotides designed based on the known sequence of single-nucleotide polymorphisms or variants of the invention. In another even more preferred embodiment, the known sequence of single-nucleotide polymorphisms or variants of the invention is the nucleotide sequence SEQ ID NO: 1, SEQ ID NO: 2, or both sequences.

Another aspect of the invention relates to a microarray, hereinafter microarray of the invention, comprising oligonucleotides or single-channel microarrays designed based on the nucleotide sequence SEQ ID NO: 1 or SEQ ID NO: 2, or any of the combinations thereof.

Therefore, for example, the oligonucleotide sequences are constructed on the surface of a chip by means of sequential elongation of a growing chain with a single nucleotide using photolithography. The oligonucleotides are thus anchored by the 3' end by means of a selective nucleotide activation method, protected by a photolabile reagent, by means of selective light application through a photomask. The photomask can be a physical or virtual photomask.

The oligonucleotide probes can therefore have between 10 and 100 nucleotides, more preferably between 20 and 70 nucleotides, and even more preferably between 24 and 30 nucleotides. For quantifying gene expression, about 40 oligonucleotides are preferably used per gene.

*In situ* synthesis on a solid support (for example glass) can be done by means of ink-jet technology, which requires longer probes. The supports could be, but without limitation, (charged) NC or nylon filters or membranes, silicon, or glass slides for microscopes covered with aminosilanes, polylysine, aldehydes or epoxy. The probe is each of the samples of the chip. The target is the sample to be analyzed: messenger RNA, total RNA, a PCR fragment, etc.

The "amplification conditions" or "extension conditions" refer interchangeably to conditions under which a polymerase can add nucleotides to the 3' end of a polynucleotide. Said amplification or extension conditions are very well known in the art (Sambrook and Russell, 2001. Molecular Cloning: A Laboratory Manual, 3rd Edition, Cold Spring Harbor Laboratory Press; Ausubel et al., Current Protocols in Molecular Biology, 1987-2007, John Wiley & Sons.)

The term "primer" used herein is known by the person skilled in the art and refers to "oligomeric compounds", mainly "oligonucleotides" although also "modified oligonucleotides", which are capable of "priming" DNA synthesis by means of a template-dependent DNA polymerase, i.e., the 3' end of the oligonucleotide, for example, provides a free 3'-OH group to which additional "nucleotides" can be bound by a template-dependent DNA polymerase, establishing a 3'-to-5' phosphodiester bond in which deoxynucleosides-triphosphate are used and in which pyrophosphate is released. Therefore, except for the intended function, there is no fundamental difference between a "primer", an "oligonucleotide" or a "probe" according to the invention. According to the invention, an "oligomeric compound" is a compound consisting of "monomeric units" which can be "nucleotides" alone or "non-natural compounds" (see below), more specifically "modified nucleotides" (or "nucleotide analogues") or "non-nucleotide compounds", alone or in combinations thereof. The "oligonucleotides" and "modified oligonucleotides" (or "oligonucleotide analogues") are sub-groups of "oligomeric compounds" in the context of the invention.

In the context of the present invention, the term "oligonucleotide" refers to "polynucleotides" formed from a plurality of "nucleotides" by way of the "monomeric unit", i.e., an "oligonucleotide" belongs to a specific sub-group of an "oligomeric compound" or "polymeric compound" of ribonucleic acid (RNA) or deoxyribonucleic acid (DNA) with "monomeric units". Phosphate groups are commonly referred to as forming the internucleoside backbone of the "oligonucleotide". The normal bond of the RNA or DNA backbone is a 3'-to-5' phosphodiester bond.

The "oligonucleotides" and "modified oligonucleotides" according to the invention can be synthesized as described primarily in the art and as is known by the person skilled in the art. The methods for preparing oligomeric compounds having specific sequences are known in the art, and they include, among others, cloning and restriction of suitable sequences and direct chemical synthesis, for example. Among others, chemical synthesis methods can include, for example, the phosphotriester method described by Narang S.A. et al., 1979, Methods in Enzymology 68,90-98, the phosphodiester method of Brown E.L. et al., 1979, Methods in Enzymology 68, 109-151, the phosphoramidite method disclosed in Beaucage et al., 1981, Tetrahedron Letters 22:1859, and the H-phosphonate method disclosed in Garegg et al., 1985, Chem. Scr. 25:280-282, and the solid support method disclosed in US patent no. 4,458,066.

As indicated above, a "nucleic acid" as well as the "target nucleic acid" is a polymeric compound consisting of "nucleotides", as known by the person skilled in the art. It is used herein to refer to a "nucleic acid" in a sample to be analyzed, i.e., the presence, absence or amount of which must be analyzed in a sample.

As it is used herein refers, the expression "complementary (nucleic acid) sequence of a nucleic acid sequence" refers to the fact that the (nucleic acid) sequence complementary to the reference sequence is the exact complement (reverse) of the nucleic acid sequence.

A nucleic acid or polynucleotide sequence can comprise the five biologically occurring bases (adenine, guanine, thymine, cytosine and uracil) and/or bases other than the five biologically occurring bases. These bases can have different purposes, for example, for stabilizing or destabilizing hybridization, for stimulating or inhibiting probe degradation, or as binding points for detectable moieties or shielding moieties. For example, a polynucleotide of the invention can contain one or more modified, non-standard, derivatized base moieties, including, but without limitation, N⁶-methyl-adenine, N⁶-tert-butyl-benzyl-adenine, imidazole, substituted imidazoles, 5-fluorouracil, 5-bromouracil, 5-chlorouracil, 5-iodouracil, hypoxanthine, xanthine, 4-acetylcytosine, 5-(carboxyhydroxymethyl)uracil, 5-carboxymethylaminomethyl-2-thiouridine, 5-carboxymethylaminomethyluracil, dihydrouracil, beta-D-galactosylqueuosine, inosine, N⁶-isopentenyladenine, 1-methylguanine, 1-methylinosine, 2,2-dimethylguanine, 2-methyladenine, 2-methylguanine, 3-methylcytosine, 5-methylcytosine, N⁶-methyladenine, 7-methylguanine, 5-methylaminomethyluracil, 5-methoxyaminomethyl-2-thiouracil, beta-D-mannosylqueuosine, 5'-methoxycarboxymethyluracil, 5-methoxyuracil, 2-methylthio-N⁶-isopentenyladenine, uracil-5-oxyacetic acid, wybutosine, pseudouracil, queuosine, 2-thiocytosine, 5-methyl-2-thiouracil, 2-thiouracil, 2-thiouracil, 4-thiouracil, 5-methyluracil (i.e., thymine), uracil-5-oxyacetic acid methyl ester, 3-(3-amino-3-N-2-carboxypropyl)uracil, (acp3)w, 2,6-diaminopurine and 5-propinyl pyrimidine. Other examples of modified, non-standard or derivatized base moieties can be found in US Patent Nos. 6,001,611, 5,955,589, 5,844,106, 5,789,562, 5,750,343, 5,728,525 and 5,679,785. Furthermore, a nucleic acid or polynucleotide sequence can comprise one or more modified sugar moieties including, but without limitation, arabinose, 2-fluoroarabinose, xylulose, and hexose.

"Hybridization melting temperature" or "Tm" refers to the temperature under specific conditions at which 50% of a polynucleotide duplex is in single-stranded form and 50% in double-stranded form. There are readily available computer programs for calculating Tm.

Another aspect of the invention relates to the use of a kit or a device of the invention or of a microarray of the invention for the prognosis of a disease occurring with sensorineural hearing loss. In a preferred embodiment of this aspect of the invention, the disease occurring with sensorineural hearing loss is Ménière's disease. In another preferred embodiment of this aspect of the invention, the sensorineural hearing loss is unilateral.

In a preferred embodiment of this aspect of the invention, the sensorineural hearing loss is selected from the list consisting of sudden hearing loss, rapidly progressive hearing loss, slowly progressive hearing loss, low frequency sensorineural hearing loss, immune-mediated inner ear disease, or any of the combinations thereof. In another preferred embodiment, the sensorineural hearing loss is furthermore associated with recurrent attack of vertigo.

Another aspect of the invention relates to a computer-readable storage medium comprising program instructions capable of making a computer run the steps of any of the methods of the invention (of the first or second method of the invention).

In a preferred embodiment of this aspect of the invention, the readable storage medium comprises at least one sequence comprised in probes of sequence SEQ ID NO: 1 and/or SEQ ID NO: 2.

In another preferred embodiment of this aspect of the invention, the readable storage medium further comprises the elements required for detecting the variants of *NFKB1.*

Another preferred embodiment of this aspect of the invention comprises oligonucleotides or single-channel microarrays designed based on at least one known sequence or an mRNA comprised in any of the probes of the invention.

Another aspect of the invention relates to a transmissible signal comprising program instructions capable of making a computer run the steps of any of the methods of the invention.

Therefore, for example, the oligonucleotide sequences are constructed on the surface of a chip by means of sequential elongation of a growing chain with a single nucleotide using photolithography. The oligonucleotides are thus anchored by the 3' end by means of a selective nucleotide activation method, protected by a photolabile reagent, by means of selective light application through a photomask. The photomask can be a physical or virtual photomask.

*In situ* synthesis on a solid support (for example glass) can be done by means of ink-jet technology, which requires longer probes. The supports could be, but without limitation, (charged) NC or nylon filters or membranes, silicon, or glass slides for microscopes covered with aminosilanes, polylysine, aldehydes or epoxy. The probe is each of the samples of the chip. The target is the sample to be analyzed: messenger RNA, total RNA, a PCR fragment, etc.

The terms "polynucleotide", "oligonucleotide" and "nucleic acid" are used interchangeably herein in reference to polymeric forms of nucleotides of any length, both ribonucleotides (RNA) and deoxyribonucleotides (DNA). The terms "amino acid sequence", "peptide", "oligopeptide", "polypeptide" and "protein" are used interchangeably herein and refer to a polymeric form of amino acids of any length, which can be chemically or biochemically modified, coding or noncoding amino acids.

Throughout the description and claims, the word "comprises" and variants thereof do not intend to exclude other technical features, additions, components or steps. For those skilled in the art, other objects, advantages and features of the invention will be inferred in part from the description and in part from the practice of the invention. The following examples and drawings are provided by way of illustration and not intended to be limiting of the present invention.

### EXAMPLES OF THE INVENTION

The invention will be illustrated below by means of assays performed by the inventors.

### Materials and Methods

### Subjects and samples

The present study included a total of 716 patients with Ménière's disease and 1628 controls from Spain. Samples were collected in 12 centers: Hospital de Poniente in El Ejido, Almeria, Hospital San Agustín in Linares, Hospital de la Fe in Valencia, Hospital Virgen de las Nieves in Granada, Hospital Universitario de Gran Canaria Dr. Negrin, Hospital Universitario in Getafe, Hospital Clínico in Santiago de Compostela, Complejo Hospitalario in Pontevedra, Clínica Universidad in Navarre, Hospital Universitario in Salamanca, Hospital de Cabueñes in Gijón, Hospital Miguel Servet in Zaragoza between January 2007 and June 2012. The patients were diagnosed after the diagnostic scale of the American Academy of Otolaryngology-Head and Neck Surgery (AAO-HNS) (Committee on Hearing and Equilibrium guidelines for the diagnosis and evaluation of therapy in Ménière's disease. American Academy of Otolaryngology-Head and Neck Foundation, Inc. Otolaryngol Head Neck Surg. 1995. 113, 181-185).

A total of 1628 samples from Spanish volunteers from the cities of Almeria, Granada, Santiago, Lugo, Oviedo and Pamplona were included as healthy controls. Quality controls were used on samples from the individuals to exclude those that did not pass quality filters (elimination of duplicated or familial samples, extreme values in the genetic structure of the population by means of principal component analysis, extreme values in heterozygosity analysis, very low call rate values).

A basic neuro-otological examination including pure-tone audiometry, nystagmus in primary position, gaze-evoked nystagmus, head-shaking nystagmus and standard caloric test was conducted on all patients. The hearing stage for each patient with definitive MD is defined as the mean of the average of four tones of 0.5, 1, 2 and 3 kHz according to the criteria of the AAO-HNS: stage 1, ≤25 dB; stage 2, 26-40dB; stage 3, 41-70dB; and stage 4, >75 dB.

The study was conducted according to the principles of the Declaration of Helsinki for research with human beings. The hospital research committee approved the research protocol. Genomic DNA was isolated from peripheral blood mononuclear cells with anticoagulants by means of the GenoVision M-48 robot (Qiagen, Venlo, Netherlands) and Qiagen MagAttract DNA Blood Mini M48 (192) kit. All the healthy patients and individuals gave their informed consent for the study.

### Genotyping by means of immunochip

The ImmunoChip is a microarray containing 196524 variants (718 insertions/deletions and 195806 SNPs). These variants are concentrated in 186 different loci which have been confirmed in earlier genomic association studies (p<10-8) in one or more autoimmune diseases. These regions contain common and rare variants (MAF<1%) and include all the known variants in the dbSNP database, the 1000 Genomes project (version from February 2010) and additional sequencing data provided by collaborators.

The samples were genotyped by means of the described Illumina 200K Infinium high-density array following the manufacturer's protocol in the Centro de Genómica e Investigación Oncológica (Genyo) Pfizer-Universidad de Granada-Junta de Andalucía.

### Genotyping quality controls

The samples were grouped by means of the Illumina Genome Studio platform algorithm. The genotypes were reviewed and validated manually and single-nucleotide polymorphisms (SNPs) with low quality tracers (call frequency <0.98, cluster separation <0.4) were eliminated. All SNPs with Gencall scores less than 0.15 were excluded. Genotyping quality controls were strict and included the elimination of SNPs with an allele frequency (MAF) of <0.05, not fitted to the Hardy-Weinberg equilibrium (HWE) <0.0001 or with differences in call rate between cases and controls of p<10⁻⁸. After quality controls, 96899 SNPs with MAF>0.05 remained.

### Statistical analysis

The genetic analysis for identifying the associated variants was performed by means of the PLINK computer program v 1.0.7. The principal component analysis (PCA) has allowed defining the groups of populations within the substructure of the analyzed samples. For this purpose, the STRUCTURE and EIGENSTRAT programs were used to define the main groups of the data, the proportions of individuals in each cluster and the marginal groups. All the individuals not grouped with the main European ancestry cluster (deviation of >3 SD from the grouped centroids) were excluded from subsequent analysis.

To evaluate statistical test inflation, after excluding the marginal groups, first the X² value of the additive genetic model is compared with its theoretical distribution using a Quantile-Quantile (Q-Q) graph to analyze the results observed against the results expected from the statistical Z-test under the null hypothesis of the absence of association throughout the genome. Data analysis is then performed. The additive model was used as a primary inference model, unless the fit testing with respect to this model was significant. In this case, the lower p-value is used for the dominant or recessive additive model.

The approach to correction by multiple comparisons combines statistical methods to reduce the rate of type I error with replication. Logistic regression analysis will be performed to determine the association using the PCA of the genotyping array and the ancestry proportion as co-variables, taking into account the structure of the population.

The first phase of these analyses was performed with the genotyping data and imputation will be used thereafter. The first imputation phase will be to complete missing genotyping data by means of the PLINK program. In a second phase, the non-genotyped variants are imputed using, as reference, data from Caucasian individuals included in the 1000 Genomes Project (CEU, GBR, IBS) updated in October 2012 and data from Caucasian individuals of HapMap3 (CEU and TSI). The accepted level of significance is p<10⁻⁸.

### Genes of the NF-kB pathway and development of hearing loss

Among the SNPs being compared, those SNPs that had been previously associated with other inflammatory or autoimmune diseases in the following genes were selected: *NFKB1* (rs3774937, rs4648011), *REL* (rs13031237), *UBE2L3* (rs5754217, rs131654, rs181362), *TNFAIP3* (rs610604) and *TNIP1* (rs2233287, rs1422673) (Table 3). To determine if any of these variants has an effect on the clinical course of the disease, the time to reach a hearing loss of more than 40 dB (hearing stage 3 or 4) was calculated for each genotype and allele by means of survival curves and log-rank test. The functional evaluation of the regulatory variants was performed *in silico* by means of HaploReg, seeQTL and RegulomeDB bioinformatics tools.

### Results

The principal component analysis (PCA) showed that the cases and controls had a similar distribution of the two principal eigenvectors in both groups, which indicates a common genetic background of the individuals in this study and the absence of stratification in the analyzed population (Figure 1).

No marker reached a significant genomic level (p<10⁻⁸) when comparing all the cases and controls. The allele frequencies of the selected *NFKB1* gene variants in patients and controls are shown in Table 4. There was no significant difference between patients with uni- or bilateral sensorineural hearing loss for any of the SNPs that were studied (p>0.05). The patients were stratified in two groups according to the presence of uni- or bilateral hearing loss and each group was compared with the controls, but none of the allelic variants reached a significant genomic level. Furthermore, none of the clinical characteristics such as migraine or autoimmune disease was significantly associated with any variant.

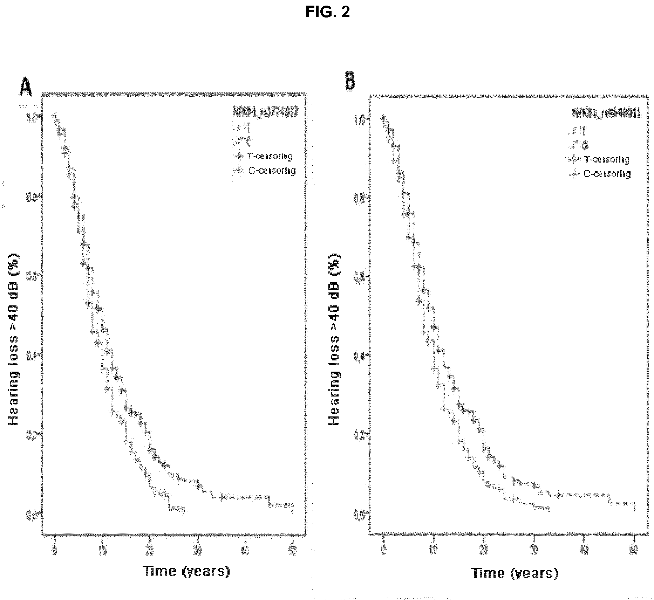

The progression of deafness in patients with uni- or bilateral sensorineural hearing loss SNHL in all the previously selected functional variants in the genes of the NF-κB pathway was also analyzed. Kaplan-Meier analysis showed that the functional variants in genes *REL, TNFAIP3, REL* and *TNIP1* have no influence on the auditory prognosis of Ménière's disease. However, two SNPs of the gene *NFKB1* (rs3774937 and rs4648011) were associated with a faster progression of hearing loss in patients with unilateral sensorineural hearing loss (2N=980). So the mean time to reach stage 3 (>40 dB HL) in patients carrying the C allele in rs3774937 and the G allele in rs4648011, respectively, decreased by 2 years (log-rank test, p=0.001 for rs3774937 and p=3.45x10⁻⁴ for rs4648011) (Figure 2). Therefore, median years to reach stage 3 were 8, 8 or 11 years from disease onset for carriers of the CC, CT or TT genotypes in rs3774937, respectively (log-rank test, p=0.002).

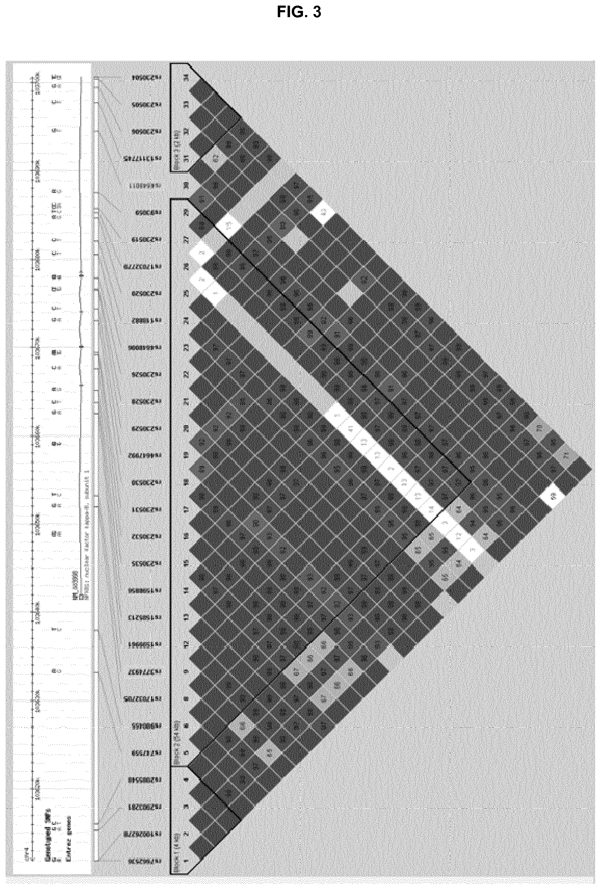

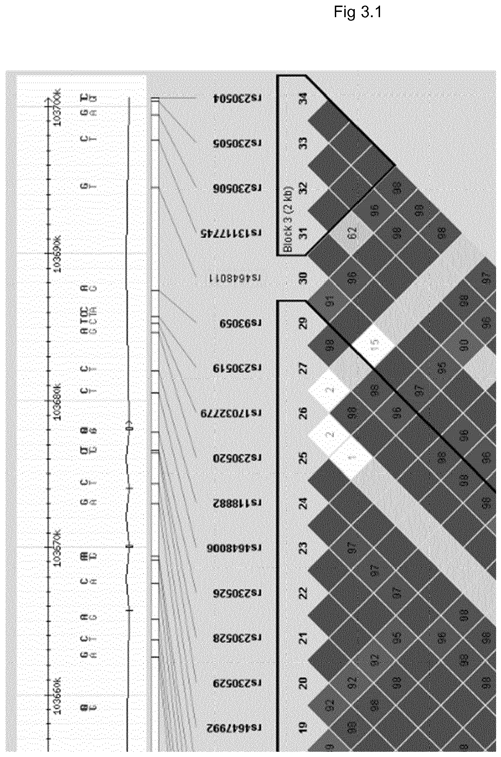

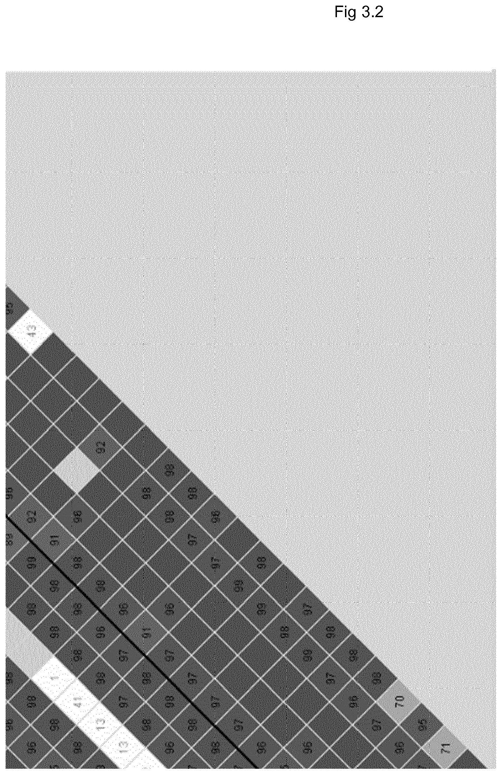

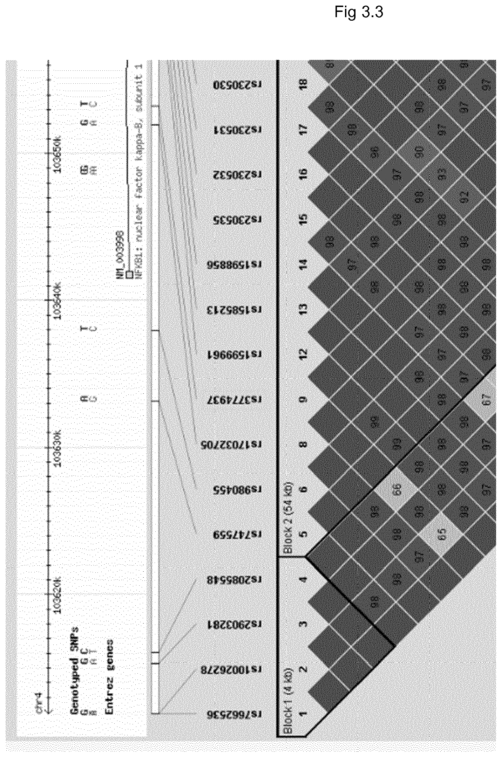

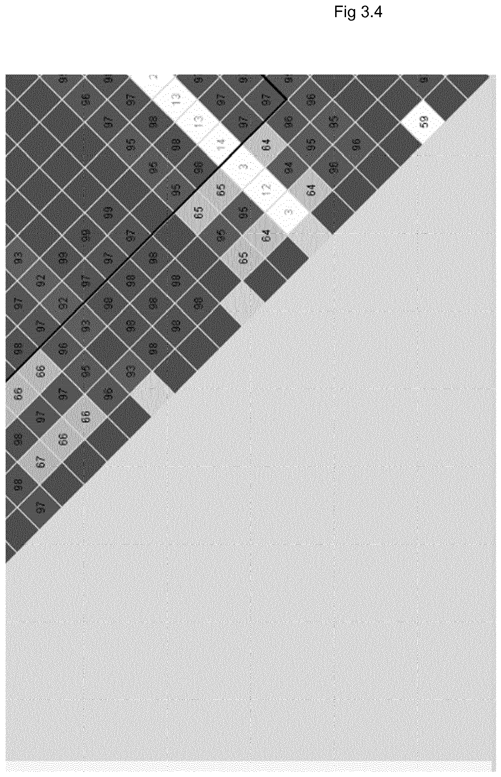

For rs4648011, the authors of the invention also found that median years to reach stage 3 were 7, 8 or 11 years from disease onset for carriers of the GG, TG or TT genotypes, respectively (log-rank test, p=0.002). Furthermore, these variants have no influence on hearing in patients with bilateral sensorineural hearing loss (p>0.05, 2N=290). The allele frequencies of rs3774937-C and rs4648011-G were 0.31 and 0.37, respectively. These variants showed a high linkage disequilibrium (r²=0.67, D'=0.95; Figure 3). The CG haplotype has a frequency of 33% and carriers of this haplotype reached 40dB 30 months before carriers of the other haplotypes (p=0.002, Table 5). Whether the carriers of the CG haplotype had a faster progression of hearing loss than patients with one of the two markers (rs3774937-C or rs4648011-G alleles) was also compared, but no additive effect was demonstrated.

Finally, *in silico* analysis of these variants predicts an interaction with the following transcription factors: DMRT1, LUN-1, YY1 for rs3774937 and Foxc1, Zfx for rs4648011.

The results show that the allelic variants of the gene *NFKB1* modify the auditory prognosis of patients with unilateral Ménière's disease. Although these markers are not associated with an increase in the susceptibility of developing Ménière's disease, these variants probably modify the interaction of *NF-kB* with other transcription factors and determined inflammatory response.

Earlier studies and these findings indicate that patients with Ménière's disease and uni- and bilateral hearing loss have a different genetic architecture and that explains why autoimmune comorbidity is more common in patients with bilateral sensorineural hearing loss and Ménière's disease.

Autoimmunity has been proposed as a mechanism in patients with bilateral Ménière's disease and although candidate gene studies found that the bilateral disease was associated with allelic variants of genes *HLA*-*DRB1* and *PTPN22*, these findings have not been replicated in the present study which includes 168 patients with bilateral Ménière's disease and is the largest sample studied up until now.

Several studies suggest the role of innate immune response (genes *MICA* and *TLR10*) in the auditory prognosis of autoimmune inner ear disease and Ménière's disease. The authors of the invention have detected that two regulatory variants of the gene *NFKB1* also have an influence on the long-term progression of hearing loss for unilateral Ménière's disease. Although the study has not found any association between markers rs3774937 and rs4648011 and Ménière's disease with unilateral hearing loss, the design has enough statistical power for detecting said association (>98% for both SNPs). However, the lack of association in patients with Ménière's disease and bilateral sensorineural hearing loss could be due to the smaller sample size in this cohort (N=168), determining a lower power for detecting a possible association (70% and 66% potential, respectively).
The bioinformatics tools used predict that those variants modify interaction with the following transcription factors: DMRT1, LUN-1, YY1 for rs3774937 and Foxc1, Zfx for rs4648011. Since the NF-κB pathway regulates inflammatory cytokine production, cell survival and plays a key role in the length of inflammatory response, these variants may have an influence on gene expression and inflammation in Ménière's disease.

Earlier studies have demonstrated that rs11096955 in the gene *TLR10* can result in susceptibility for patients with bilateral sensorineural hearing loss in patients with Ménière's disease. TLRs are one of the main defense mechanisms in infectious and non-infectious diseases and an unsuitable activation of the TLR pathways has been associated with several autoimmune diseases.

TLRs activate an intracellular signal via MyD88 triggering a complex cascade including the IRAK1-IRAK4-TRAF6, TAK1-TAB1-TRAF6-UBC13 and IKK complexes. This signal leads to the induction of a large number of pro-inflammatory genes by means of the NF-κB transcription factor. Functional variants of genes *NFKB1, REL, UBE2L3, TNFAIP3* and *TNIP1* were selected for evaluating their prognostic potential for Ménière's disease. Generally, the data indicates that allelic variants in some genes of the innate immune response such as *TLR10* and *NFKB1* may act as regulatory genes capable of modifying the clinical progression of hearing loss in Ménière's disease.

**Table 3: Associations described in the allelic variants in the NF-kB pathway**

| **Chrom** | **Position^{a}** | **rsID** | **Gene (variant)** | **Phenotype** | **p-value** |
|---|---|---|---|---|---|
| **6** | 138199417 | rs610604 | TNFAIP3 (intron) | Psoriasis | 5.53x10-5 |
| **5** | 150440097 | rs2233287 | TNIP1(intron) | Asthma, systemic sclerosis | 0.039, 6.17x10-4 |
| **5** | 150438988 | rs1422673 | TNIP1(intron) | Asthma | 0.011 |
| **2** | 61136129 | rs13031237 | REL (intron) | Rheumatoid arthritis | 7.29x10-3 |
| **22** | 21939675 | rs5754217 | UBE2L3 (intron) | Lupus | 0.012 |
| **22** | 21917190 | rs131654 | UBE2L3 (intron) | Lupus | 1.12x10-7 |
| **22** | 21932068 | rs181362 | UBE2L3 (intron) | HDL cholesterol | 3.72x10-4 |
| **4** | 103434253 | rs3774937 | NFKB1 (intron) | Body weight | 0.041 |
| **4** | 103475444 | rs4648011 | NFKB1 (intron) | Body weight | 0.040 |

| | | | | | |
|---|---|---|---|---|---|
| ^{a} NCBI constructed human genome, version 37 ^{b} http://www.ncbi.nlm.nih.gov/gap | | | | | |

**Table 4:Allele frequencies of the SNPs of the gene NFKB1 in patients and controls**

| **SNP** | **POSITION** | **CONTROL** | **A1/A2** | **UNILATERAL** | **OR(95%Cl)** | **P** | **BILATERAL** | **OR(95% Cl)** | **P** |
|---|---|---|---|---|---|---|---|---|---|
| | | | | | 0.942 (0.817- | | | 0.859 (0.681- | |
| **rs6533014** | Chr4:103349740 | 0.491 | G/A | 0.476 | 1.088) 1.024 (0.860- | 0.418 | 0.453 | 1.083) 1.000 (0.755- | 0.197 |
| **rs6845971** | Chr4:103359744 | 0.214 | A/G | 0.218 | 1.217) 1.041 (0.897- | 0.791 | 0.214 | 1.325) 0.814 (0.635- | 0.998 |
| **rs17032705** | Chr4:103432974 | 0.361 | A/G | 0.370 | 1.207) 0.970 (0.832- | 0.594 | 0.315 | 1.043) 0.736 (0.567- | 0.103 |
| **rs3774937** | Chr4:103434253 | 0.323 | C/T | 0.317 | 1.131) 1.043 (0.898- | 0.700 | 0.260 | 0.956) 0.882 (0.691- | 0.021 |
| **rs4648011** | Chr4:103475444 | 0.363 | G/T | 0.373 | 1.210) 0.918 (0.754- | 0.531 | 0.335 | 1.126) 1.213 (0.904- | 0.315 |
| **rs13117745** | Chr4:103478703 | 0.164 | A/G | 0.152 | 1.118) 0.888 (0.730- | 0.397 | 0.192 | 1.628) 0.792 (0.568- | 0.196 |
| **rs4648090** | Chr4:103527068 | 0.167 | A/G | 0.151 | 1.082) 0.958 (0.718- | 0.239 | 0.137 | 1.105) 0.674 (0.393- | 0.169 |
| **rs4648128** | Chr4:103536024 | 0.067 | G/A | 0.064 | 1.279) 0.897 (0,702- | 0.775 | 0.046 | 1.155) 1.031 (0.705- | 0.148 |
| **rs230547** | Chr4:103536261 | 0.100 | A/G | 0.091 | 1.147) | 0.386 | 0.103 | 1.507) | 0.875 |

**Table 5: Effect of rs3774937 (T>C) and rs4648011 (T>G) haplotypes on hearing loss in patients with Ménière's disease. The time to reach hearing of >40 dB was compared by survival curves by means of the Kaplan-Meier method.**

| **Time to reach >40 dB (years, mean ± SD)** | | | |
|---|---|---|---|
| HAPLOTYPE (rs3774937, rs4648011) | FREQUENCY (%) | UNILATERAL | BILATERAL |
| CG | 33 | 8 ± 0.48 | 10 ± 0.90 |
| GT | 5 | 8 ±1.12 | 12 ± 1.20 |
| TT | 62 | 10 ± 0.47 | 12 ± 0.67 |

## Claims

1. Use of the gene *NFKB1* for obtaining data useful for the prognosis of a disease occurring with sensorineural hearing loss.

2. Use of single-nucleotide polymorphisms or variants rs3774937 and/or rs4648011 of the gene *NFKB1* according to the preceding claim.

3. Use of the gene *NFKB1* according to claim 1 or of single-nucleotide polymorphisms or variants rs3774937 and/or rs4648011 of the gene *NFKB1* according to claim 2, wherein the disease occurring with sensorineural hearing loss is Ménière's disease or autoimmune inner ear disease.

4. Use of the gene *NFKB1* according to the preceding claims, wherein the sensorineural hearing loss is unilateral.

5. A method for obtaining data useful for the prognosis of a disease occurring with sensorineural hearing loss, comprising:
a) obtaining a biological sample isolated from an individual, and
b) detecting single-nucleotide polymorphisms or variants rs3774937 and/or rs4648011 in the isolated biological sample of (a).

6. A method for the prognosis of a disease occurring with sensorineural hearing loss, which comprises steps (a) and (b) according to claim 3 and further comprises:
c) including the individual from (a) in the group of individuals with rapid progression of the disease occurring with sensorineural hearing loss when said individual has a lower homozygous allele frequency for rs3774937 and/or rs4648011.

7. The method according to any of claims 5-6, wherein the disease occurring with sensorineural hearing loss is Ménière's disease or autoimmune inner ear disease.

8. The method according to any of claims 5-7, wherein the isolated sample is genomic DNA obtained from peripheral blood.

9. The method according to any of claims 5-8, wherein the individual belongs to a population of European descent, and more preferably of Spanish descent.

10. The method according to any of claims 5-9, wherein the sensorineural hearing loss is unilateral.

11. A method for classifying an individual suffering or who has the probability of suffering from a disease occurring with sensorineural hearing loss, preferably unilateral sensorineural hearing loss, and more preferably Ménière's disease or autoimmune inner ear disease, in one of two groups, wherein group 1 comprises individuals who can be identified by means of the method according to claims 5-10, and wherein group 2 represents the remaining individuals.

12. Use of a pharmaceutical composition comprising gentamicin in the preparation of a medicinal product for the treatment of an individual from group 1 suffering or who has the probability of suffering from a disease occurring with sensorineural hearing loss, preferably unilateral sensorineural hearing loss, and more preferably Ménière's disease or autoimmune inner ear disease, who can be identified by means of the method of claim 11.

13. A kit or device comprising at least one oligonucleotide complementary to any of the sequences SEQ ID NO: 1 and/or SEQ ID NO: 2.

14. A microarray comprising oligonucleotides or single-channel microarrays designed based on the nucleotide sequence SEQ ID NO: 1 and/or SEQ ID NO: 2.

15. Use of a kit or a device according to claim 13 or of a microarray according to claim 14 for the prognosis of a disease occurring with sensorineural hearing loss.

16. Use of a kit or a device according to the preceding claim, wherein the disease occurring with sensorineural hearing loss is Ménière's disease or autoimmune inner ear disease.

17. Use of a kit or a device according to any of claims 15-16, wherein the disease occurring with sensorineural hearing loss is Ménière's disease.

18. Use of a kit device according to claims 15-16, wherein the sensorineural hearing loss is unilateral.
